# EUROPEAN PATENT APPLICATION

(11) **EP 3 130 313 A1**
(43) Date of publication of application: **15.02.2017**
(21) Application number: 16184916.1
(22) Date of filing: 16.10.2009
(51) Int. Cl.: A61F 2/12, A61B 5/055, A61B 5/00

(54) **METHOD OF DETECTING RUPTURES IN A PROSTHETIC IMPLANT**

(30) Priority: 17.10.2008 US 106458
(62) Divisional of application: 14155727.2
(71) Applicant: ALLERGAN, INC., Irvine, CA 92612 (US)
(72) Inventor: Yacoub, Kerim, Solvang, CA 93463 (US); Powell, Thomas E., Santa Barbara, CA California 93111 (US)
(74) Representative: Hoffmann Eitle

(57) **Abstract**

The present invention provides a method of detecting whether an implanted fluid-filled prosthetic implant has ruptured, the method comprising the steps of imaging the implant using magnetic resonance imaging and inspecting the magnetic resonance image for defects in the implant, wherein the implant comprises a shell which comprises at least one layer containing a matrix material and an additive distributed therein, the density of the additive being greater than that of the matrix material.

## Description

### Field of the Invention

The present invention relates to prosthetic implants, for example, mammary implants.

### Background

Implantable prostheses are commonly used to replace or augment body tissue. In the case of breast cancer, it is sometimes necessary to remove some or all of the mammary gland and surrounding tissue that creates a void that can be filled with an implantable prosthesis. The implant serves to support surrounding tissue and to maintain the appearance of the body. The restoration of the normal appearance of the body has an extremely beneficial psychological effect on post-operative patients, eliminating much of the shock and depression that often follows extensive surgical procedures. Implantable prostheses are also used more generally for restoring the normal appearance of soft tissue in various other areas of the body.

Fluid filled implants can be imaged and evaluated in vivo using mammography, magnetic resonance imaging (MRI), ultrasonography and computer tomography (CT). MRI is one of the most accurate imaging technologies available for breast implants. However, there remains a need for technologies which afford more accurate diagnostic imaging of fluid filled implants.

### Summary

The present invention provides prosthetic implants, for example, fluid filled prosthetic implants and flexible shells of such implants which have enhanced visibility when viewed using conventional imaging technologies, for example, magnetic resonance imaging techniques. The implants may be mammary implants.

In one aspect of the invention, implants are provided which generally comprise a flexible shell and a soft filler material, for example, a silicone based gel, enclosed by the shell.

In one embodiment, the shell comprises a composition more visible to magnetic resonance imaging than conventional fluid filled implant shells. Particularly, in one embodiment, the shell comprises a matrix material, for example, an elastomeric material, such as a silicone elastomeric material, and a secondary additive distributed in the matrix material. In one embodiment, the secondary additive comprises a material having a density greater than the density of the matrix material. For example, the matrix material may comprise a silicone elastomer and the additive may comprise a biocompatible metal, for example, a biocompatible metal oxide dispersed throughout the silicone elastomer.

In one embodiment, the additive is a metal selected from the group of metals consisting of aluminum, brass, titanium, Nitinol (nickel-titanium alloy), steel, alloys and mixtures thereof.

In an exemplary embodiment, the additive is a metal oxide having a density of about 4.0 g/ml. More specifically, the additive may be titanium oxide (TiO₂). Even more specifically, the concentration of TiO₂ by weight is between about 0.5% and about 25%. In one embodiment, the concentration of TiO₂ in the shell is about 8%.

In another embodiment of the invention, an implant shell is provided having a specific gravity of at least 1.15 or greater. For example, in some embodiments, the specific gravity of the shell is greater than about 1.20, for example, greater than about 1.40.

In another aspect of the invention, methods of forming soft prosthetic implants and flexible shells of such implants are provided. In one embodiment, a method of the invention comprises, in part, providing a dispersion comprising an elastomer and a metal oxide and forming an implant shell from the dispersion. In some embodiments, the invention comprises providing a quantity of a matrix material, for example, a silicone elastomer, mixing into the matrix material a quantity of a secondary additive having a density greater than the density of the matrix material, and forming a dispersion from the mixture.

In yet another aspect of the invention, a method of detecting a rupture in a fluid-filled prosthetic implant is provided. The method generally comprises providing a fluid-filled flexible implant having a shell wall including at least one layer comprising a matrix material and an additive distributed therein having a density greater than the matrix material. In some embodiments, the shell comprises a silicone elastomer matrix material and a metal oxide dispersed therein. The method further comprises the step of imaging the implanted fluid-filled prosthetic implant using magnetic resonance imaging (MRI) and inspecting the magnetic resonance image for contrasts, irregularities, discontinuities and/or other possible indications of defects, for example, rupture or potential rupture in the shell of the implant

In yet another aspect of the invention, prosthetic implants are provided comprising a shell including laser etched indicia. For example, in one embodiment, a prosthetic implant shell including indicia formed of fused titanium from TiO₂ in the material which makes up the shell is provided. The indicia may be in the form of a label indicating batch number, manufacturer, location of manufacture, model or style number, trademark, and/or other useful information relating to the implant. In one embodiment the indicia comprises a label in the form of a bar code, for example, etched by laser on an exterior surface of the shell.

In another aspect of the invention, a method of manufacturing a fluid filled implant having indicia thereon is provided. In one embodiment, the method comprises providing a flexible implant shell including at least one layer comprising a matrix material and a metal oxide distributed in the matrix material. In one embodiment, the metal oxide is titanium dioxide. The method further comprises providing indicia on the shell by using a laser to inscribe the indicia in the shell, wherein the metal oxide reacts to the laser, for example, becomes fused, to form visible indicia in the shell. The indicia may be in the form of a label indicating batch number, manufacturer, location of manufacture, model or style number, trademark, and/or other useful information relating to the implant. In one embodiment the indicia comprises a label in the form of a bar code, for example, etched by laser on an exterior surface of the shell.

### Brief Description of the Drawing

Features and advantages of the present invention will become appreciated as the same become better understood with reference to the specification, claims, and appended drawing of which:

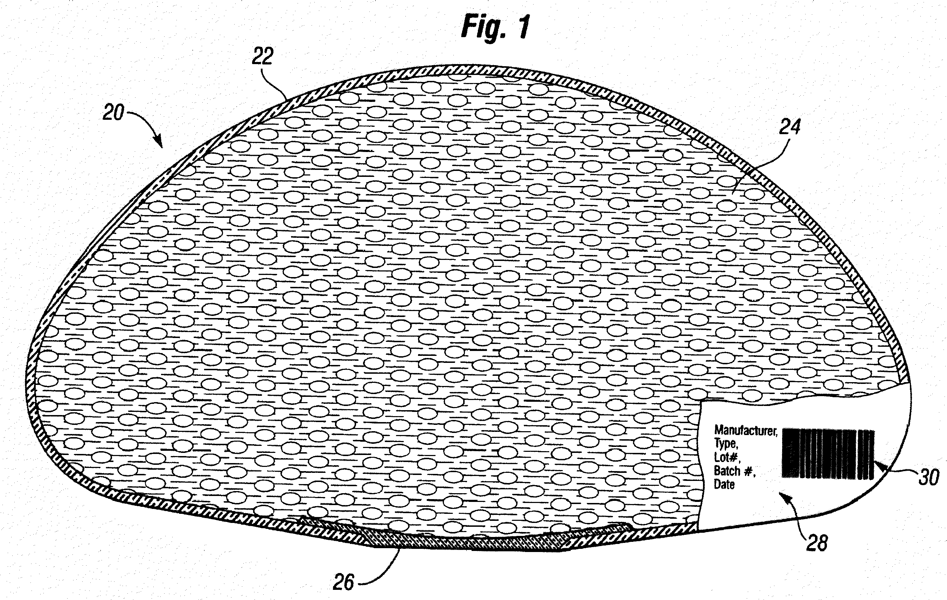

Figure 1 is a cross-sectional view through a fluid-filled prosthetic implant in accordance with an embodiment of the present invention.

### Detailed Description

The present application provides prosthetic implants and shells for such prosthetic implants. The implants may be mammary implants useful for reconstruction or augmentation of the breast.

The implants of the invention generally comprise a core material for example, a core material of a silicone gel and an elastomer shell enclosing the core material. The shell may comprise a matrix material, typically a silicone elastomer, and an additive distributed within the matrix material, the additive causing the shell to have an increased density relative to an otherwise identical shell without the additive. For example, in some embodiments of the invention, the shell comprises a silicone elastomer matrix and the additive comprises a material having a greater density than the silicone elastomer matrix material such that the shell has an increased density relative to a substantially identical shell made of silicone elastomer and not including the additive.

In some embodiments, the shells of the present invention provide enhanced visibility when imaged in vivo using conventional imaging techniques, relative to conventional silicone elastomer shells not including the additive. For example, the present implants and shells thereof are more readily visible in magnetic resonance images of the implant.

In one aspect of the invention, the shell is made up of a material having a density greater than the density of body tissue, for example, breast tissue, adjacent the implant when the implant has been implanted in a patient.

Specific gravity is defined as the ratio of the density of a given solid or liquid substance to the density of water at 4°C (39°F). At this temperature, the density of pure water is about 1.0 g/ml (about 62.4 lb/ft³). Materials with a specific gravity greater than 1.0 have a higher density than pure water at 4°C. For practical purposes, the density of a material in g/ml (or g/cc) is equivalent to the specific gravity of that material when water is used as the reference density.

Conventional silicone elastomer implant shells have a specific gravity close to that of water, and typically no greater than about 1.10 or about 1.15. In contrast, shells of some embodiments of the present invention have a specific gravity of greater than 1.15. For example, some of the implant shells in accordance with the present invention have a specific gravity of greater than about 1.20, for example, greater than about 1.40, for example, greater than about 1.60, for example, greater than about 1.80, or more.

In one embodiment, the additive comprises a non-ferromagnetic or weakly ferromagnetic material. The additive may comprise a biocompatible metal, for example, a metal oxide.

The material may comprise, for example, a metal selected from aluminum, brass, titanium, titanium alloys, Nitinol (nickel-titanium alloy), stainless steel and blends thereof. In some embodiments, the additive is a metal oxide, specifically a non-ferromagnetic or weakly ferromagnetic metal oxide. In one embodiment, the additive is titanium dioxide (TiO₂).

The concentration of additive, for example, TiO₂, in the shell may be in a range of between about 0.5% and about 25% by weight, for example, between about 5% and about 10% by weight. In one embodiment, the concentration of TiO₂ in the shell is about 8% by weight.

The shell may comprise a single, unitary layer comprising the matrix material and additive as described elsewhere herein. In other embodiments, the implant shell comprises a plurality of such layers of material, wherein at least one of said layers comprises the matrix material and additive. In some embodiments of the invention, the shell has a tensile strength comparable to or greater than a tensile strength of an identical shell comprising the matrix material without said additive dispersed therein.

In one embodiment of the invention, the additive is added to a dispersion of the matrix material in the form of a paste or powder. The powder or paste may comprise the additive formulated with a resin, for example, a silicone fluid-resin. The desired concentration of additive in the final mixture may be calculated based on percent solids of silicone elastomer (rubber) in the batch of dispersion to the amount of additive added thereto. In a specific embodiment, an additive comprising a metal oxide, for example, TiO₂ is initially provided in a powder form and then is converted to a paste form by mixing said powder with a suitable polymer or other material. The additive "paste" is then combined with a liquid form of silicone elastomer including an appropriate solvent. The combining can be accomplished by mixing or other suitable technique to ensure substantially uniform distribution of the additive in the silicone elastomer. The liquid silicone elastomer/titanium dioxide dispersion is used to form the shells of the implants of the present invention, for example, using conventional dip-molding or rotational molding techniques known to those of skill in the art.

Fig. 1 illustrates an exemplary breast implant 20 of the present invention, the implant comprising a shell 22 comprising a matrix material and an additive distributed therein having a density greater than a density of the matrix material, such that the density of the shell material is greater than about 1.2 g/ml, for example, is greater than about 1.4 g/ml.

The implant 20 also comprises a fluid 24 enclosed by the shell 22. The fluid may be a silicone gel, saline or other appropriate prosthetic implant filler material. In some embodiments, the flush patch 26 covers a manufacturing hole formed on the shell during molding thereof. In other embodiments, the implant may include a fluid adjustment valve.

As described elsewhere herein, the shell 22 comprises the matrix material, for example, a commercially available silicone elastomer used for forming conventional implant shells , and an additive distributed therein, for example, TiO₂.

In one embodiment of the invention, the shell 22 includes marking, labeling or other indicia 28, formed on the shell 22 by contacting the shell with focused electromagnetic energy, for example, in the form of a laser, which causes discoloration of the additive component of the shell. In one embodiment, the additive is titanium dioxide and the indicia is formed of fused titanium.

For example, in one embodiment of the invention, the shell comprises a matrix material and an additive which facilitates marking of the shell, for example, when the shell is contacted with radiation, for example, ultraviolet, visible, or near infrared radiation, or other radiation form which will react with the additive to form the indicia without compromising the integrity (e.g. strength) of the shell. Such energy source can be supplied by a conventional laser source. For example, in one embodiment of the invention, the additive comprises titanium dioxide and the shell includes marking, labeling or other indicia 28 of titanium, formed on the shell by reaction of focused energy with the titanium dioxide component of the shell. In one embodiment, the indicia is a computer-readable marking, for example, in the form of a bar code which provides information about the shell.

Marking of the shell may be achieved by moving a laser beam over a portion of the shell using conventional beam steering methods, by moving the shell in relation to the laser beam and/or by masking the shell and applying light energy to an unmasked portion of the shell.

Suitable lasers for use in accordance with the present invention include, for example, neodymium:yttrium aluminum garnet (Nd:YAG) lasers, carbon dioxide(CO₂) lasers, diode lasers, excimer lasers and the like.

Conventional YAG lasers emit light in the near-infrared spectrum at wavelengths of 1064 nm. Such lasers typically have continuous power outputs of from about 1 watt to about 50 watts, and can be operated in a pulsed mode at typical peak powers of from about 1 watt to about 45 kilowatts. For pulsed mode operation, frequencies of from about 1 to about 64,000 pulses/second may be used.

Suitable lasers for marking the shells of the present invention include EPILOG Legend Model 6000 L24EX-30W, EPILOG Helix Model 8000. Suitable software for use with this equipment includes CADLink Engravelab 5.0 software. Another suitable laser is Electrolox Razor 30 W razor with Scriba3 software which can interact with Oracle and generate data for the marking of the shell.

In accordance with one embodiment of the present invention, the size of the laser spot that impinges the shell may have a diameter of between about 0.1 micron to about 500 microns, or greater.

It will be appreciated that the laser parameters may be controlled in order to provide sufficient localized radiation to create titanium-based marking from the titanium dioxide in the shell while avoiding damage to the integrity of the shell.

In some embodiments movement of the laser beam is controlled by a computer which may be used to create the indicia.

Alternatively or additionally, the additive may comprise an additive other than titanium dioxide, for example, another suitable metal oxide, that is biocompatible and reacts with focused energy applied to the shell containing the additive to produce visible marking on the shell.

In a related embodiment of the invention, methods for making a prosthetic implant having indicia are provided. For example, the method comprises the steps of providing a flexible implant shell including a matrix material and an additive dispersed within the matrix material, forming indicia on the shell by applying electromagnetic energy to the shell and causing the additive to react with the energy, for example, become discolored by the energy.

In one embodiment, the laser-inscribed indicia are detectable on the shell in vivo, for example, using MRI or other suitable imaging technique. For example, on an MRI image, the implant itself will be visible and, in addition, the indicia thereon will also be detectable, for example, distinctly visible and/or otherwise readable.

Alternatively, or in addition, the same information may be incorporated into a computer readable bar code 30 that makes automatic identification though a scanner possible. The inclusion of a non-ferromagnetic or weakly ferromagnetic metal such as TiO₂ in the shell 22 enhances the visibility of such a label, as the metal at the surface fuses to create visible lines without weakening the shell material.

Although the invention has been described and illustrated with a certain degree of particularity, it is to be understood that the present disclosure has been made only by way of example, and that numerous changes in the combination and arrangement of parts can be resorted to by those skilled in the art without departing from the scope of the invention, as hereinafter claimed.

Embodiments:
1. A shell for a fluid-filled prosthetic implant, comprising:
   a flexible implant shell having a shell wall including at least one layer comprising a matrix material and an additive distributed therein having a density greater than the matrix material, such that the specific gravity of the shell is greater than about 1.20.
2. The shell of 1, wherein the specific gravity of the shell is greater than about 1.40.
3. The shell of 1, wherein the matrix material is a silicone elastomer and the additive is a non-ferromagnetic or weakly ferromagnetic metal.
4. The shell of 2, wherein the additive is selected from the group consisting of:
   aluminum,
   brass,
   titanium,
   a titanium alloy,
   nickel-titanium alloy, and
   a stainless steel.
5. The shell of 2, wherein the additive is titanium dioxide (TiO₂).
6. The shell of 5, wherein the concentration by weight of TiO₂ in the shell is between about 0.5% and about 25%.
7. The shell of 6, wherein the concentration by weight of TiO₂ in the shell is about 8%.
8. A shell for a fluid-filled prosthetic implant, comprising:
   a flexible implant shell having a shell wall including at least one layer comprising a matrix material and titanium dioxide (TiO₂) distributed therein, wherein the concentration by weight of TiO₂ in the shell is between about 0.5% and about 25%.
9. The shell of 8, wherein the concentration by weight of TiO₂ in the shell is about 8%.
10. The shell of 8, wherein the matrix material comprises a silicone elastomer.
11. The shell of 8, having a specific gravity of greater than about 1.20.
12. The shell of 8, having a specific gravity of greater than about 1.40.
13. A method of forming a soft prosthetic implant shell, comprising:
   providing a quantity of a matrix material in bulk form;
   combining the matrix material with a quantity of an additive in bulk form having a density greater than the matrix material, to form a dispersion; and
   forming an implant shell from the dispersion, the specific gravity of the shell being greater than about 1.20.
14. The method of 13, wherein the additive is TiO₂.
15. The method of 14, wherein the concentration by weight of TiO₂ in the shell is between about 0.5% and about 25%.
16. The method of 15, wherein the concentration by weight of TiO₂ in the shell is about 8%.
17. A method of detecting ruptures in a fluid-filled prosthetic implant, comprising the steps of:
   providing a fluid-filled flexible implant having a shell comprising a matrix material and an additive distributed therein having a density greater than the matrix material, such that the specific gravity of the shell is greater than about 1.20;
   inserting the fluid-filled prosthetic implant into a body cavity; and
   imaging the fluid-filled prosthetic implant *in vivo* with magnetic resonance imaging (MRI) and inspecting for discontinuities in the shell image.
18. The method of 17, wherein the additive is TiO₂.

## Claims

1. A method of detecting whether an implanted fluid-filled prosthetic implant has ruptured, the method comprising the steps of imaging the implant using magnetic resonance imaging and inspecting the magnetic resonance image for defects in the implant, wherein the implant comprises a shell which comprises at least one layer containing a matrix material and an additive distributed therein, the density of the additive being greater than that of the matrix material.

2. A method according to Claim 1, wherein the additive comprises a metal oxide.

3. A method according to Claim 2, wherein the metal oxide is titanium oxide.

4. A method according to Claim 3, wherein titanium oxide is contained in the shell in an amount in the range of 5-10 wt.%.

5. A method according to any preceding claim, wherein the matrix material is a silicone elastomer.

6. A method according to any preceding claim, wherein the shell has a specific gravity greater than 1.20.

7. A method according to Claim 6, wherein the shell has a specific gravity greater than 1.40.
